# EUROPEAN PATENT APPLICATION

(11) **EP 1 348 963 A1**
(43) Date of publication of application: **01.10.2003**
(21) Application number: 02007114.8
(22) Date of filing: 28.03.2002
(51) Int. Cl.: G01N 33/68

(54) **Schizophrenia related gene**

(71) Applicant: Mitsubishi Pharma Corporation, Osaka-shi, Osaka 541-0046 (JP)
(72) Inventor: Cochran, Susan, Mitsubishi Pharma Corp. Tokyo H.O., Tokyo 103-8405 (JP); Yamagami, Keiju, Mitsubishi Pharma Corp. Tokyo H.O, Tokyo 103-8405 (JP); Ohashi, Yoshitaka, Mitsubishi Pharma. Corp. Tokyo, Chuo-ku, Tokyo 103-8405 (JP)
(74) Representative: von Kreisler, Alek, Dipl.-Chem.

(57) **Abstract**

The present invention provides a new use of the polynucleotide of SEQ ID NO:1 or fragments thereof, or the polypeptide derived from that polynucleotide in the field of the schizophrenia.

## Description

The present invention relates to the identification of the Kv3.1 α-subunit gene postulated to be involved in and/or associated with schizophrenia. The present invention also relates to its use in the therapy and/or diagnosis of schizophrenia and to a method of screening for a compound which regulates the expression of the gene or the activity of the Kv3.1 channel.

Schizophrenia is a devastating mental illness that affects 1% of the world population, the aetiology of which remains elusive. To date, there is a poor understanding of the genes involved.

One of the goals of modern antipsychotic drug development is to produce a drug which is more effective in ameliorating the negative symptoms and cognitive deficits characteristic of schizophrenia than existing therapies. Although typical and atypical antipsychotic drugs, such as haloperidol and clozapine respectively, are effective in attenuating the positive symptoms, they are ineffective (haloperidol) or minimally effective (clozapine) against the negative symptoms and cognitive dysfunction associated with the disease (Goldberg, T.E. et al. British Journal of Psychiatry, 1993, 162; 43-48). The development of improved antipsychotic drugs that will have superior action against the negative symptoms and cognitive dysfunction has been severely hampered by the lack of knowledge of which genes are involved and/or associated with schizophrenia. Moreover little is known about the genes, or more specifically any alteration of expression or mutation of the genes in a patient suffering from schizophrenia.

The voltage-gated potassium channel 3.1 (Kv3.1, the first member of the Shaw-related subfamily of voltage-gated potassium channels) is predominantly located within a subset of GABAergic interneurons within the brain, namely parvalbumin-containing interneurons (Weiser et al., The Journal of Neuroscience, March 1994, 14(3); 949-972). Its function within these interneurons is to allow fast repolarisation of the cells and thus, contributes to the fast-firing phenotype of these neurons (Du et al. The Journal of Neuroscience, January 15, 1996, 16(2); 506-518, Chow et al., The Journal of Neuroscience, November 1, 1999,_19(21); 9332-9345; Erisir et al., The Journal of Neurophysiology, 1999, 82; 2476-2489). Parvalbumin expression has been shown to be altered within the prefrontal cortex in schizophrenia (Beasley and Reynolds, Schizophrenia Research, 1997, 24; 349-355), however Kv3.1 expression has not been investigated in this disease state.

The voltage-gated potassium channel 3.1 is known to have many names such as Kv3.1, KShIIIB, NGK2-Kv4, NGK2, Kv4 or KCNC1. In the present specification, the inventors use the name Kv3.1 for the voltage-gated potassium channel 3.1. Two alternate splice variants of the Kv3.1 α-subunit gene exist (Kv3.1a and Kv3.1b), with each variant differing only in their intracellular C-terminal sequence in the Kv3.1 α-subunit.

The human Kv3.1 α-subunit gene has also been cloned (GenBank accession number NM_004976; Localization of a highly conserved human potassium channel gene (NGK2-Kv4; KCNC1) to chromosome 11p15. Reid T; Rudy B; Vega-Saenz de Miera E; Lau D; Ward DC and Sen K. (1993). Genomics 15 (2), 405-411). It displays ∼99% homology to the rat sequence, and there is >90% homology between rat and mouse sequences.

Chronic treatment with phencyclidine (hereinafter PCP) in the rat provides an animal model of schizophrenia with superior face, construct and predictive validity (WO01/75440). It has been shown to mimic metabolic changes (metabolic hypofunction in the prefrontal cortex, thalamus and auditory structures) that are observed in schizophrenic patients, that can be correlated to both positive and negative symptomology and cognitive deficits associated with the disease, and also neurochemical changes observed in postmortem tissue from schizophrenic patients such as decreased parvalbumin expression and decreased 5HT2A binding. Parvalbumin mRNA was selectively decreased within certain brain regions in the chronic PCP rat model of schizophrenia, the prelimbic region of the prefrontal cortex and the reticular nucleus of the thalamus and this effect was modulated by chronic antipsychotic treatment. Due to the high degree of co-localization between parvalbumin and the Kv3.1 channel, the aim was to determine whether Kv3.1 α-subunit expression is similarly altered by the same chronic PCP rat model of schizophrenia.

The present inventors have found that the Kv3.1 α-subunit gene, or a nucleotide having SEQ ID NO:1, shows altered expression in the brain of the chronic PCP rat model of schizopherenia, and also found that the nucleotide and the protein that this nucleotide encodes are useful for the diagnosis or treatment of schizopherenia. The present invention also provides a method of screening a compound that regulates expression and activity of this nucleotide and/or protein. Accordingly, the present invention provides the following.
(1) Use of the polynucleotide of SEQ ID NO:1 or fragments thereof, or the polypeptide derived from that polynucleotide for diagnosing schizophrenia.
(2) A method of diagnosing schizophrenia, said method comprising using the polynucleotide of SEQ ID NO:1 or fragments thereof, or the polypeptide derived from that polynucleotide as an indicator of schizophrenia.
(3) Use of the polynucleotide of SEQ ID NO:1 or fragments thereof, or the polypeptide derived from that polynucleotide in the identification of compounds which modulate the expression of said polynucleotide.
(4) Use of the polypeptide derived from the polynucleotide of SEQ ID NO:1 or fragments thereof, in the identification of compounds which modulates the activity of Kv3.1 channel.
(5) A method for screening a compound which regulates the expression of the polynucleotide of SEQ ID NO:1, which comprises:
   (a) bringing a test compound into contact with a cell which enables expression of the polynucleotide of SEQ ID NO:1,
   (b) detecting the expression of the polypeptide derived from the polynucleotide in said cell, and
   (c) selecting a compound which promotes or suppresses the induction of the polypeptide in comparison with a control (vehicle).

The present invention relates to methods for prognostic and/or diagnostic evaluation of schizophrenia and/or for the identification of subjects who are predisposed to schizophrenia, for example by examination of allelic variation of the gene identified herein in an individual or by determination of the expression of the gene in an individual. Furthermore, the invention provides methods for evaluating the efficacy of drugs for such disorders, and monitoring the progress of patient symptoms involved in clinical trials for the treatment of such disorders.

The invention further provides methods for the identification of compounds that modulate the expression of the Kv3.1 α-subunit gene and/or the activity of the Kv3.1 channel. Such identified compounds may be used in the treatment of schizophrenia.

By the "modulate" is meant here the action to promote or suppress the expression of the gene, which varies according to the desired effect. In consideration of the application to Schizophrenia, however, screening of a compound that increases the level of expression of Kv3.1 α-subunit gene is preferable.

There is also provided a method for screening a compound which regulates the expression of the polynucleotide of SEQ ID NO:1, which comprises:
(a) bringing a test compound into contact with a cell which enables expression of the polynucleotide of SEQ ID NO:1,
(b) detecting an expression of the polypeptide derived from the polynucleotide in said cell, and
(c) selecting a compound which promotes or suppresses the induction of the polypeptide in comparison with a control (vehicle).

In the above-mentioned screening method, the "test compound" is free of any particular limitation as long as it is the object compound for which capability of regulating the expression of the polynucleotide of SEQ ID NO:1 is desired to be determined, and it may be a novel or known compound. A combination drug can be the object compound. More specifically, chronic PCP administration that affects the expression of Kv3.1 α-subunit mRNA is combined with administration of the test compound. By analyzing the metabolic and neurochemical state induced by the chronic PCP administration and changes in the expression of Kv3.1 α-subunit mRNA, the effect of the test compound can be investigated.

In the above-mentioned screening method, the "cell which enables expression of the polynucleotide of SEQ ID NO:1" is free of any particular limitation as long as it possibly expresses the polynucleotide of SEQ ID NO:1. It may be naturally occurring or processed to express said polynucleotide by genetic recombination.

The information obtained through the screening method and diagnostic method of the present invention (hereinafter to be generally referred to as characterization) can suggest relevant methods for the treatment or control of schizophrenia. For example, relevant treatment can include a modulation of gene expression and/or gene product activity. Characterization procedures such as those described herein can indicate whether such modulation should be positive or negative. As used herein, "positive modulation" refers to an increase in gene expression or the activity of gene product of interest. "Negative modulation", as used herein, refers to a decrease in gene expression or activity.

*In vitro* systems can be designed to identify compounds capable of binding with said Kv3.1 α-subunit gene product of the invention.

SEQ ID NO:1 shows the nucleotide sequence of the rat Kv3. 1 α-subunit gene of the present invention which was observed to be differentially expressed in the brain in the chronic PCP rat model of schizophrenia.

SEQ ID NO:2 shows the amino acid sequence of the rat Kv3. 1 α-subunit of the present invention which was observed to be differentially expressed in the brain in the chronic PCP rat model of schizophrenia.

SEQ ID NO:3 shows the oligonucleotide (45mer) used for *in situ* hybridization for the Kv3.1 α-subunit mRNA.

The present invention is explained in more detail in the following by way of Experiments, which are not to be construed as limitative.

In this specification, Y931 means 8-fluoro-12-(4-methylpiperazin-1-yl)-6H-[1]benzothieno[2,3-b][1,5]benzo-diazepine monomaleate, which is known as an atypical antipsychotic drug (WO99/116479).

### Experiment

### Method

Male Sprague-Dawley rats, weighing 224-260g at the beginning of the study, were randomly assigned to one of ten treatment groups as follows: vehicle (0.02N HCl in saline)-vehicle (polyethyleneglycol); PCP-vehicle; PCP-Y931 0.3 mg/kg/day; PCP-Y931 1 mg/kg/day; PCP-Y931 3 mg/kg/day; PCP-Y931 10 mg/kg/day; PCP-olanzapine 0.3 mg/kg/day; PCP-olanzapine 1 mg/kg/day; PCP-olanzapine 3 mg/kg/day; PCP-olanzapine 10 mg/kg/day. The dose of PCP used was 2.24 mg/kg (free base equivalent, equal to 2.58 mg/kg PCP.HCl) and PCP was administered according to the chronic PCP rat model of schizophrenia (WO01/75440) Briefly, i.p. injections of PCP (2.24 mg/kg) or saline (1 ml/kg) were administered once daily on days 1-5, 8,10,12,15,17,19,22,24,26. Osmotic minipumps (2ML4 (28day), Alzet) were implanted subcutaneously on day 8 prior to PCP injection to deliver doses of antipsychotics or vehicle detailed above for 21 days of drug treatment. The osmotic minipumps were primed *in vitro* for 24 hours in order to ensure pumping started immediately upon implantation.

29 days after the first PCP injection, the animals were sacrificed, the brains removed and stored at -70°C. Osmotic minipumps were also removed and the dose administered was verified. The brains were sectioned at -20°C using a cryostat (Leica, CM1850), with 14 µm coronal sections being thaw-mounted onto silanized slides (Dako, Japan) from the following bregma levels according to the Rat Brain Atlas, Paxinos and Watson (2000): 3.22 mm; 1.6 mm, -1.80 mm and -4.8 mm. The sections were then stored at -80°C over silica gel until required. The oligonucleotide (45mer) used for *in situ* hybridization for the Kv3.1 α-subunit mRNA was 5'-ATAGTCGAAGTGGCTGTGGGCGTCCGGCTCCGCCAGCCAGGCAAG-3' (SEQ ID NO:3) complementary to bases 1261-1305 of the rat Kv3.1 α-subunit (GenBank Accession Number NM_012856; Luneau et al., Proc. Natl. Acad. Sci. USA. 1991, 88; 3932-3936). This probe sequence recognizes both alternate splice variants (Kv3.1a and Kv3.1b) of the Kv3.1 channel. The probe was 3' end-labeled with terminal deoxyribonucleotidyl transferase (Boehringer Mannheim) and with the isotope 5-α-³⁵S-dATP (Amersham, specific activity > 1000 Ci/mmol) and incubated at 37°C for one hour. The reaction was terminated by addition of 40 µl diethyl pyrocarbonate (DEPC)-treated water. The probe was purified using the QIAquick nucleotide removal kit (Qiagen). The extent of probe labeling was assessed using β-scintillation counting, and probes labeled from 100,000 to 300,000 d.p.m. µl⁻¹ were used for *in situ* hybridization. On the day of the *in situ* hybridization experiment the sections were fixed in 4% paraformaldehyde-0.1M phosphate buffer for 30 mins at 4°C, rinsed twice for 5 mins in 0.1M phosphate buffer saline (PBS), acetylated with 0.25% acetic anhydride in 0.1M triethanolamine for 10mins, rinsed twice for 5 mins in 2X SSC (0.15M NaCl, 15 mM sodium citrate), dehydrated and delipidated through a graded series of ethanol and chloroform. The sections were then air dried and then hybridized overnight at 42 °C with 0.2-1x10⁻⁶d.p.m. of the labeled probe in 100 µl of hybridization solution (Hybrisol 1, Intergen) containing 0.1M dithiothreitol. After hybridization, the sections were washed twice for 10 secs in 1xSS at 55°C, four times for 15 mins in 1xSS at 55°C, for 1 hour in 1xSS at room temperature and twice in water for 5 mins. The sections were then dipped successively in 60%, 80%, 90%, 95% and 100% ethanol and air dried. The sections were exposed to BAS 5000 image plates and developed after 7 days. Kv3.1 α-subunit mRNA expression was quantified using a computer-based densitometry system (MCID 5, Canada). Statistical analysis of the data was performed using a students t-test (vehicle-vehicle vs PCP-vehicle) followed by Dunnett's Method (PCP-vehicle vs PCP-antipsychotics). Statistical significance was defined as p<0.05.

### Results

Table 1 shows the effect of chronic PCP and antipsychotic treatment on Kv3.1 α-subunit mRNA expression. Chronic PCP treatment selectively decreased Kv3.1 α-subunit mRNA expression within the prelimbic region of the prefrontal cortex (PrL, -31%), the dorsal part of the reticular nucleus of the thalamus (dRt, -15%) and the dorsolateral striatum (DLStr, -16%). Within the PrL, both Y931 and olanzapine, at all doses administered, significantly reversed the PCP-induced decrease back to control levels. However, within the dRt and the DLStr, neither Y931 nor olanzapine significantly modulated the PCP-induced effect.

Compared to PCP-vehicle, PCP-olanzapine treatment (1 mg/kg/day, only) produced a significant decrease in Kv3.1 α-subunit mRNA expression within the granule cell layer of the dentate gyrus (DGgcl) and a significant increase in Kv3.1 α-subunit mRNA expression within the ventromedial striatum (VMStr) (0.3 & 3 mg/kg/day olanzapine, only).

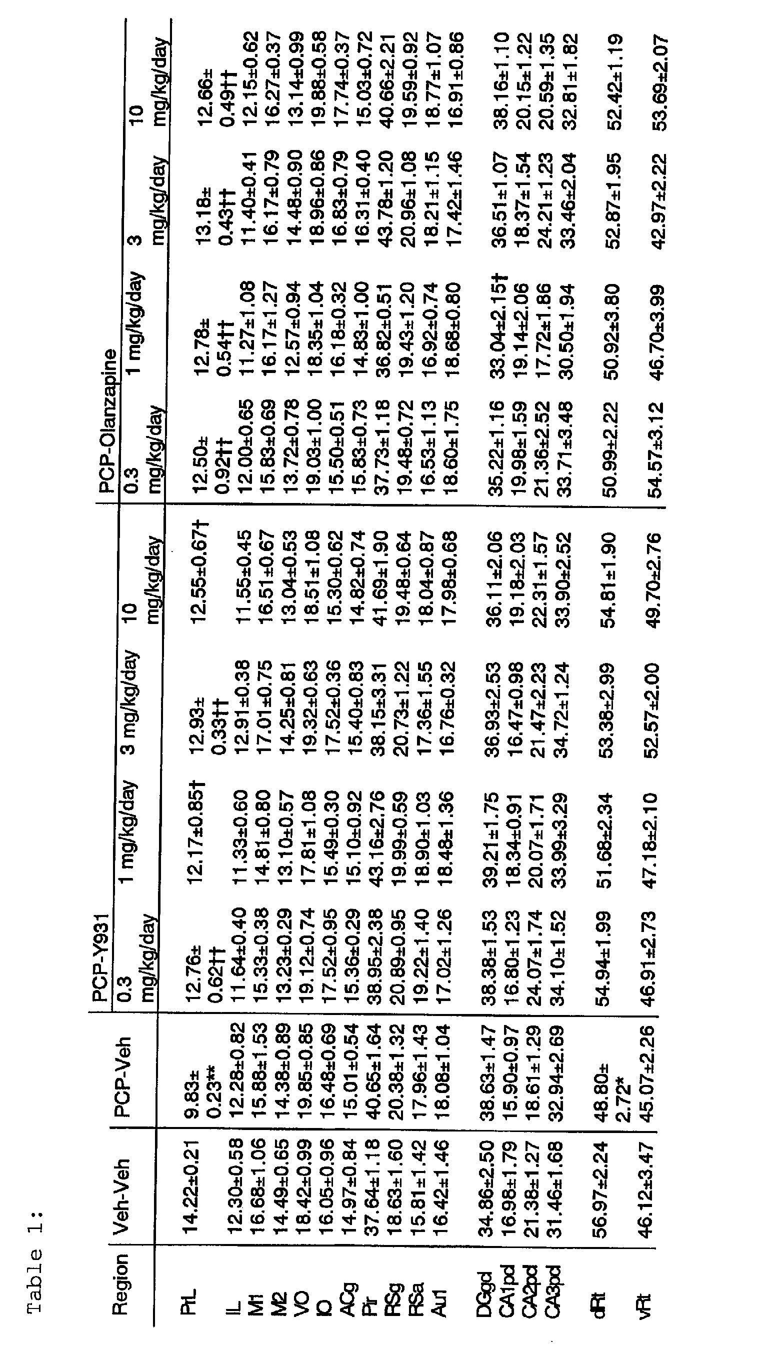

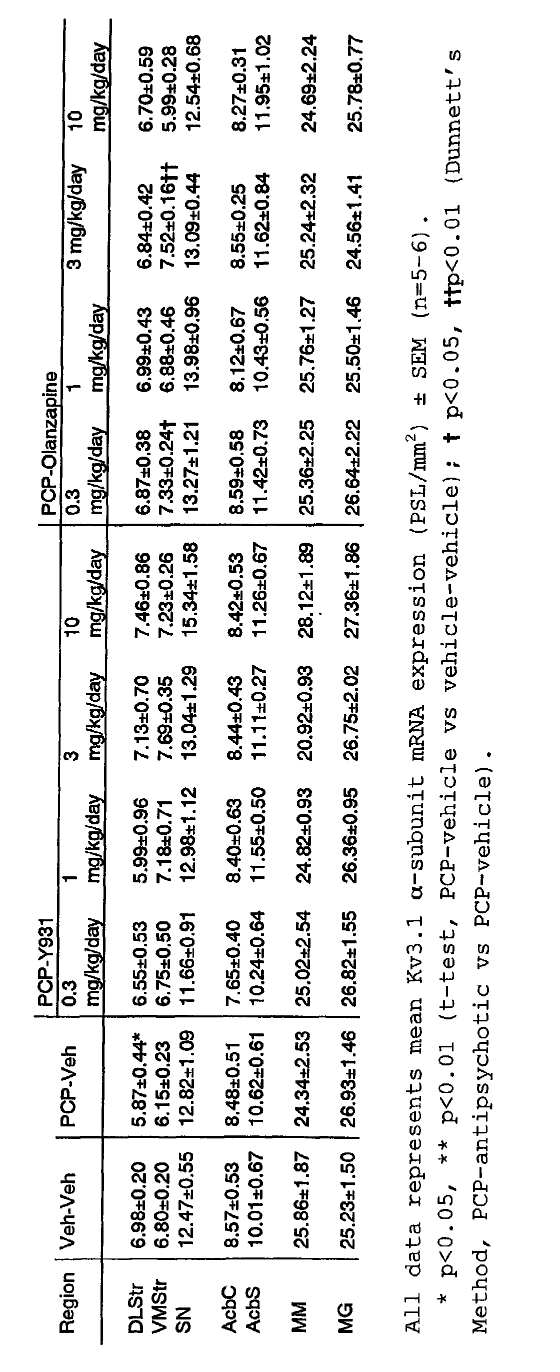

### Abbreviations:

PrL, prelimbic cortex;
IL, infralimbic cortex;
M1, primary motor cortex;
M2, secondary motor cortex;
vO, ventral orbital cortex;
1O, lateral orbital cortex;
Acg, anterior cingulate cortex;
Pir, piriform cortex;
RSg, granular retrosplenial cortex;
Rsa, agranular retrosplenial cortex;
Au1, primary auditory cortex;
DGgcl, granule cell layer of the dentate gyrus;
CA1-CA3 pcl, pyramidal cell layer of the CA1-CA3 fields of the hippocampus; dRt, vRt, dorsal and ventral parts of the reticular nucleus of the thalamus.
DLStr, dorsolateral striatum;
VMStr, ventromedial striatum;
SN, substantia nigra;
AcbC, nucleus accumbens core;
AcbS, nucleus accumbens shell;
MM, mammillary body;
MG, medial geniculates.

The decrease in Kv3.1 α-subunit mRNA expression observed in this study parallel the selective changes previously observed in parvalbumin mRNA expression within the prelimbic cortex and the dorsal nucleus of the reticular nucleus of the thalamus. Moreover, the PCP-induced decreases in Kv3.1 α-subunit mRNA expression were reversed by chronic coadministration of Y931 and olanzapine, as were PCP-induced changes in parvalbumin mRNA expression. Since parvalbumin expression is altered within the prefrontal cortex of schizophrenic patients and in rats after treatment with chronic PCP, these data suggest that Kv3.1 α-subunit mRNA may also be altered within schizophrenia in a similar manner.

A drug acting at the Kv3.1 channel has a potential therapeutic role in Schizophrenia. The hypothesis is that the decrease in Kv3.1 may affect the firing activity of the GABAergic interneurons in which it is located, which further contributes to an imbalance between the excitatory (glutamatergic) and inhibitory (GABAergic) pathways within the prefrontal cortex (which may be linked to negative symptoms and cognitive dysfunction). A drug which can restore Kv3.1 expression may restore GABAergic interneuron activity back to normal.

Kv3.1 may be a good marker for the screening of antipsychotic activity because of the reversal with the atypical antipsychotic olanzapine and Y931 as shown in these data.

## Claims

1. Use of the polynucleotide of SEQ ID NO:1 or fragments thereof, for diagnosing schizophrenia.

2. Use of the polypeptide derived from the polynucleotide of SEQ ID NO:1 or fragments thereof, for diagnosing schizophrenia.

3. A method of diagnosing schizophrenia, said method comprising using the polynucleotide of SEQ ID NO:1 or fragments thereof, as an indicator of schizophrenia.

4. A method of diagnosing schizophrenia, said method comprising using the polypeptide derived from the polynucleotide of SEQ ID NO:1 or fragments thereof, as an indicator of schizophrenia.

5. Use of the polynucleotide of SEQ ID NO:1 or fragments thereof, in the identification of compounds which modulate the expression of said polynucleotide.

6. Use of the polypeptide derived from the polynucleotide of SEQ ID NO:1 or fragments thereof, in the identification of compounds which modulate the expression of said polypeptide.

7. Use of the polypeptide derived from the polynucleotide of SEQ ID NO:1 or fragments thereof, in the identification of compounds which modulates the activity of Kv3.1.

8. A method for screening a compound which regulates an expression of the polynucleotide of SEQ ID NO:1, which comprises:
(a) bringing a test compound into contact with a cell which enables expression of the polynucleotide of SEQ ID NO:1,
(b) detecting an expression of the polypeptide derived from the polynucleotide in said cell, and
(c) selecting a compound which promotes or suppresses the induction of the polypeptide in comparison with a control (vehicle).


SEQUENCE LISTING

<110> Mitsubishi Pharma Corporation
<120> SCHIZOPHRENIA RELATED GENE
<130> 08981

<160> 3

<170> PatentIn version 3.1

<210> 1
<211> 3977
<212> DNA

<213> Rattus norvegicus

<220>
<221> CDS
<222> (1162)..(2919)
<223>

<220>
<221> gene
<222> (1)..(3977)
<223>

<220>
<221> polyA_signal
<222> (3958)..(3963)
<223>

<300>
<301> Luneau, C. J., Williams, J. B., Marshall, J., Levitan, E. S., Oliva, C.,
Smith, J. S., Antanavage, J., Folander, K., Stein, R. B., Swanson, R.,
Kaczmarek, L. K. and Buhrow, S. A.
<302> Alternative splicing contributes to K+ channel diversity in the
mammalian central nervous system.
<303> Proc. Natl. Acad. Sci. USA.
<304> 88
<306> 3932-3936

<308> NM_012856

<400> 1
gaacgagcaa gcgagcaggc agcggagcag cagcccggag gcagcagcgg cggcggtggt 60

ggcggcagtg gccgctccgc gcctcgcctc cctggcctcg ccagcctcgt tgcgccgcgc 120

ccggacctgc cacccccgcc taccgggccc gacctcggca gcgcccctcg catcggctgc 180

cagcgccacc cgccctccca gagcagtgag cggagtgcgg ggcgcagaaa caccggagcc 240

agtgcccgcc agccccgcca agcaaaacgg gctgagagaa cctacgctcg gcccagcgcg 300

gctccacagc cgtccgggag ctgtcccttc agcaccgccg cggaagcctg agtcccagcg 360

cagcccagcg gaacccctga cccagcccga gcgcaccagg cgctgcgctt agcggcagcc 420

ccgcggggag caccettgtg cccctaccca ggggacctgc catctcctcc gggaggcggg 480

gagaaggaac gaggagggag gtcgggcagc ttcacccgcc accgagggga gtaggcagac 540

aagcaaggag atccgaaggg gatcggtcac gcagggagga gagcaagccg catccgtccc 600

caccgcaccg ggagcgcgct gcggcgacca ccgcagagcc aagcgcggat ttctcgtaac 660

cccctctttc ctagcagggg ctggtctatc ggtccggccc ccgaaacgga gcagctgacc 720

ttcccctaaa aagctttgtg tcgatttctc catttttccc gctgagatgg taccgggagc 780

ccggcccccc catcatctct cccctcctcc cgggcctcgc cgctgagcgc cgccccactc 840

ccttcctccc tccctcttta cctccctcct ctcttccact gcctcctccg cagccaggcc 900

agctccctct cacactaggc gccttaaaga ccctaaggag cccgcgcaca cacccctgga 960

cccgcacctg actgacagct ccccctgagg gggcttggct cgttggttgg ggtgggtggc 1020

cagagccgcg gtcctctgtg ccccccgacg gctgggggga ggggggaaga ggccctgcgc 1080

ccccctcccc gtcgccaact ccccctggcg gcagctccca tgggtgtcgc tgggccgcgc 1140

catgcctaag gggggcgccgc g atg ggc caa ggg gac gag agc gag cgc atc 1191
Met Gly Gln Gly Asp Glu Ser Glu Arg Ile
1               5                   10

gtg atc aac gtg ggc ggc acg cgc cac cag acg tac cgc tcg acg ctg 1239
Val Ile Asn Val Gly Gly Thr Arg His Gln Thr Tyr Arg Ser Thr Leu
                15                  20                  25

cgc acg ctg ccc ggc acg cgg ctt gcc tgg ctg gcg gag ccg gac gcc 1287
Arg Thr Leu Pro Gly Thr Arg Leu Ala Trp Leu Ala Glu Pro Asp Ala
            30                  35                  40

cac agc cac ttc gac tat gac ccg cgc gct gac gag ttc ttc ttc gac 1335
His Ser His Phe Asp Tyr Asp Pro Arg Ala Asp Glu Phe Phe Phe Asp
        45                  50                  55

cgc cac ccg ggc gtc ttc gct cac atc ctg aac tat tac cgc acc ggc 1383
Arg His Pro Gly Val Phe Ala His Ile Leu Asn Tyr Tyr Arg Thr Gly
    60                  65                  70

aag cta cac tgc ccg gcc gac gtg tgc ggg ccg ctc tac gag gag gag 1431
Lys Leu His Cys Pro Ala Asp Val Cys Gly Pro Leu Tyr Glu Glu Glu
75                  80                  85                  90

ctg gcc ttc tgg ggc atc gac gag acg gac gtg gag ccc tgc tgc tgg 1479
Leu Ala Phe Trp Gly Ile Asp Glu Thr Asp Val Glu Pro Cys Cys Trp
                95                  100                 105

atg acg tat cgc cag cac cgc gac gct gag gag gcg ctg gac agc ttt 1527
Met Thr Tyr Arg Gln His Arg Asp Ala Glu Glu Ala Leu Asp Ser Phe
            110                 115                 120

ggt ggc gcg ccc ttg gac aac agc gcc gac gac gcg gac gcc gac ggc 1575
Gly Gly Ala Pro Leu Asp Asn Ser Ala Asp Asp Ala Asp Ala Asp Gly
        125                 130                 135

ccc ggc gac tcg ggc gac ggc gag gac gag ctg gag atg acc aag aga 1623
Pro Gly Asp Ser Gly Asp Gly Glu Asp Glu Leu Glu Met Thr Lys Arg
    140                 145                 150

ttg gcg ctc agt gac tcc cca gat ggc cgg cct ggc ggc ttc tgg cgc 1671
Leu Ala Leu Ser Asp Ser Pro Asp Gly Arg Pro Gly Gly Phe Trp Arg

155                 160                 165                 170

cgc tgg cag cca cgc atc tgg gca ctg ttc gag gac ccc tac tca tcc     1719
Arg Trp Gln Pro Arg Ile Trp Ala Leu Phe Glu Asp Pro Tyr Ser Ser
                175                 180                 185

cgc tat gcg cgg tac gtg gcc ttt gcc tcc ctc ttc ttc atc ctg gtc     1767
Arg Tyr Ala Arg Tyr Val Ala Phe Ala Ser Leu Phe Phe Ile Leu Val
            190                 195                 200

tcc atc aca acc ttc tgt ctg gag acc cac gag cgc ttc aac ccc atc     1815
Ser Ile Thr Thr Phe Cys Leu Glu Thr His Glu Arg Phe Asn Pro Ile
        205                 210                 215

gtg aac aag aca gaa att gag aac gtt cga aat ggc aca caa gtg cgg     1863
Val Asn Lys Thr Glu Ile Glu Asn Val Arg Asn Gly Thr Gln Val Arg
    220                 225                 230

tac tac cgg gaa gcg gag acg gag gcc ttc ctc acc tac atc gag ggt     1911
Tyr Tyr Arg Glu Ala Glu Thr Glu Ala Phe Leu Thr Tyr Ile Glu Gly
235                 240                 245                 250

gtc tgc gtg gtc tgg ttc acc ttc gag ttc ctc atg cgt gtt gtc ttc     1959
Val Cys Val Val Trp Phe Thr Phe Glu Phe Leu Met Arg Val Val Phe
                255                 260                 265

tgt ccc aac aag gtg gag ttc atc aag aac tcc ctc aat atc att gac     2007
Cys Pro Asn Lys Val Glu Phe Ile Lys Asn Ser Leu Asn Ile Ile Asp
            270                 275                 280

ttc gtg gcc atc ctc ccc ttc tac ctg gag gtg ggc cta agt ggc ctg     2055

Phe Val Ala Ile Leu Pro Phe Tyr Leu Glu Val Gly Leu Ser Gly Leu
        285                 290                 295

tcc tcc aaa gct gcc aag gac gtg cta ggc ttc ctg cgc gtc gtg cgc 2103
Ser Ser Lys Ala Ala Lys Asp Val Leu Gly Phe Leu Arg Val Val Arg
    300                 305                 310

ttc gtg cgc atc ctc cgt atc ttc aag ctg acc cgc cat ttt gtg ggc 2151
Phe Val Arg Ile Leu Arg Ile Phe Lys Leu Thr Arg His Phe Val Gly
315                 320                 325                 330

ctg cgg gtc ctg ggc cac acg ctc cgt gcc agc acc aac gag ttc ctg 2199
Leu Arg Val Leu Gly His Thr Leu Arg Ala Ser Thr Asn Glu Phe Leu
                335                 340                 345

ctg ctt atc atc ttc ctg gcc ctg ggt gtg ctc atc ttt gcc acc atg 2247
Leu Leu Ile Ile Phe Leu Ala Leu Gly Val Leu Ile Phe Ala Thr Met
            350                 355                 360

atc tac tat gct gag agg ata ggg gca cag ccc aat gac ccc agc gcc 2295
Ile Tyr Tyr Ala Glu Arg Ile Gly Ala Gln Pro Asn Asp Pro Ser Ala
        365                 370                 375

agc gaa cac aca cac ttt aaa aat atc ccc atc ggc ttc tgg tgg gct 2343
Ser Glu His Thr His Phe Lys Asn Ile Pro Ile Gly Phe Trp Trp Ala
    380                 385                 390

gtg gtc acc atg acg aca ctg ggc tat gga gac atg tat cct cag acg 2391
Val Val Thr Met Thr Thr Leu Gly Tyr Gly Asp Met Tyr Pro Gln Thr
395                 400                 405                 410

tgg tct ggg atg ttg gtg gga gca ctg tgc gcc ctg gct ggt gtg ctg 2439
Trp Ser Gly Met Leu Val Gly Ala Leu Cys Ala Leu Ala Gly Val Leu
                415                 420                 425

acc att gcc atg ccg gtg ccc gtc atc gtg aac aat ttt ggg atg tac 2487
Thr Ile Ala Met Pro Val Pro Val Ile Val Asn Asn Phe Gly Met Tyr
            430                 435                 440

tac tct tta gcc atg gct aag cag aaa cta cca aag aaa aaa aag aag 2535
Tyr Ser Leu Ala Met Ala Lys Gln Lys Leu Pro Lys Lys Lys Lys Lys
        445                 450                 455

cat att ccg cgg cca cca cag ctg gga tct ccc aat tat tgt aaa tct 2583
His Ile Pro Arg Pro Pro Gln Leu Gly Ser Pro Asn Tyr Cys Lys Ser
    460                 465                 470

gtc gta aac tct cca cac cac agt act cag agt gac aca tgt ccg ctg 2631
Val Val Asn Ser Pro His His Ser Thr Gln Ser Asp Thr Cys Pro Leu
475                 480                 485                 490

gcc cag gaa gaa att tta gaa att aac aga gca gat tcc aaa ctg aat 2679
Ala Gln Glu Glu Ile Leu Glu Ile Asn Arg Ala Asp Ser Lys Leu Asn
                495                 500                 505

ggg gag gtg gcg aag gcc gcg ctg gcg aac gaa gac tgc ccc cac ata 2727
Gly Glu Val Ala Lys Ala Ala Leu Ala Asn Glu Asp Cys Pro His Ile
            510                 515                 520

gac cag gcc ctc act ccc gat gag ggc ctg ccc ttt acc cgc tcg ggc 2775
Asp Gln Ala Leu Thr Pro Asp Glu Gly Leu Pro Phe Thr Arg Ser Gly
        525                 530                 535

acc cgc gag aga tac gga ccc tgc ttc ctc tta tca acc ggg gag tac 2823
Thr Arg Glu Arg Tyr Gly Pro Cys Phe Leu Leu Ser Thr Gly Glu Tyr
    540                 545                 550

gcg tgc ccg cct ggt gga gga atg aga aag gat ctt tgc aaa gaa agc 2871
Ala Cys Pro Pro Gly Gly Gly Met Arg Lys Asp Leu Cys Lys Glu Ser
555                 560                 565                 570

cct gtc att gct aag tat atg ccg aca gag gct gtg aga gtg act tga 2919
Pro Val Ile Ala Lys Tyr Met Pro Thr Glu Ala Val Arg Val Thr
                575                 580                 585

ccaggcggct tggccgagga cactggtggc tattaagcat ctgggtggac ctgcagcccc 2979

tcctcaccct cggacagagt aaattcacgc catgcaggtt tgccggacga gtccgagtgg 3039

ccccgggcat tgtactaaga cggacgtagc tttttctacg gcccacggta actgaccgta 3099

gaggattcgc ccttctttct tttgattttc tttttctttc tttttttttt ttttagaagt 3159

taccttttat ttggggaggg ggggtcaagg ggagccttag catgacttgc atgaagttaa 3219

acagaaaacc cagctaaacc aaccccacca gcccctgctg tgactgtatg ctcactctaa 3279

ccaccaccga gaaccagaga aaggcaagag tctcttattt cttccagcat tctttgacac 3339

gtagtgtcgg agtcaaactg tgacggcgct tggtagaacc ctgtacattt ccgggagtgg 3399

gtgcgggggg gggggggagg tcagggacta aggatggggc caggggagtg gattgctttt 3459

ttgtacacga gacccagagg aaaggtccca aaaaggcagt cagtcatcgg tcacattgac 3519

ctcaccttca tagctcacct ctcgctcgga aaaccaagaa ctatgctccg aataggattg 3579

tggaaattca cactgccacc ctcttccacc tccgtttggc atgcttgcga cccaatggac 3639

ctctccccaa gaccccgaca gcggctagtt taggtcaatt ctctcatctt ggtgtgtagc 3699

tccagtgtga ccaagtttca taacaaattg tttcatagtt acaccacatg gattttccaa 3759

tgtcatttta aagccaggat gtagagtccc tccagttcag gaccacccag aggggcgcct 3819

cccctccacc tttactaagg cgaaaaccga cactgtatgc aatttagtac ttcagagctc 3879

aaagaaaaaa aaaaaaagaa aaaaaaagaa aaaagaaaaa ctgcatgccc aatgttatgc 3939

aaagagattc gagcatgaaa taaattttgt aacatggt 3977

<210> 2
<211> 585
<212> PRT
<213> Rattus norvegicus

<400> 2

Met Gly Gln Gly Asp Glu Ser Glu Arg Ile Val Ile Asn Val Gly Gly
1               5                   10                  15

Thr Arg His Gln Thr Tyr Arg Ser Thr Leu Arg Thr Leu Pro Gly Thr
            20                  25                  30

Arg Leu Ala Trp Leu Ala Glu Pro Asp Ala His Ser His Phe Asp Tyr
        35                  40                  45
Asp Pro Arg Ala Asp Glu Phe Phe Phe Asp Arg His Pro Gly Val Phe
    50                  55                  60
Ala His Ile Leu Asn Tyr Tyr Arg Thr Gly Lys Leu His Cys Pro Ala
65                  70                  75                  80
Asp Val Cys Gly Pro Leu Tyr Glu Glu Glu Leu Ala Phe Trp Gly Ile
                85                  90                  95
Asp Glu Thr Asp Val Glu Pro Cys Cys Trp Met Thr Tyr Arg Gln His
            100                 105                 110
Arg Asp Ala Glu Glu Ala Leu Asp Ser Phe Gly Gly Ala Pro Leu Asp
        115                 120                 125
Asn Ser Ala Asp Asp Ala Asp Ala Asp Gly Pro Gly Asp Ser Gly Asp
    130                 135                 140
Gly Glu Asp Glu Leu Glu Met Thr Lys Arg Leu Ala Leu Ser Asp Ser
145                 150                 155                 160
Pro Asp Gly Arg Pro Gly Gly Phe Trp Arg Arg Trp Gln Pro Arg Ile
                165                 170                 175
Trp Ala Leu Phe Glu Asp Pro Tyr Ser Ser Arg Tyr Ala Arg Tyr Val
            180                 185                 190
Ala Phe Ala Ser Leu Phe Phe Ile Leu Val Ser Ile Thr Thr Phe Cys

        195                 200                 205

Leu Glu Thr His Glu Arg Phe Asn Pro Ile Val Asn Lys Thr Glu Ile
    210                 215                 220

Glu Asn Val Arg Asn Gly Thr Gln Val Arg Tyr Tyr Arg Glu Ala Glu
225                 230                 235                 240

Thr Glu Ala Phe Leu Thr Tyr Ile Glu Gly Val Cys Val Val Trp Phe
                245                 250                 255

Thr Phe Glu Phe Leu Met Arg Val Val Phe Cys Pro Asn Lys Val Glu
            260                 265                 270

Phe Ile Lys Asn Ser Leu Asn Ile Ile Asp Phe Val Ala Ile Leu Pro
        275                 280                 285

Phe Tyr Leu Glu Val Gly Leu Ser Gly Leu Ser Ser Lys Ala Ala Lys
    290                 295                 300

Asp Val Leu Gly Phe Leu Arg Val Val Arg Phe Val Arg Ile Leu Arg
305                 310                 315                 320

Ile Phe Lys Leu Thr Arg His Phe Val Gly Leu Arg Val Leu Gly His
                325                 330                 335

Thr Leu Arg Ala Ser Thr Asn Glu Phe Leu Leu Leu Ile Ile Phe Leu
            340                 345                 350

Ala Leu Gly Val Leu Ile Phe Ala Thr Met Ile Tyr Tyr Ala Glu Arg
        355                 360                 365

Ile Gly Ala Gln Pro Asn Asp Pro Ser Ala Ser Glu His Thr His Phe
    370                 375                 380

Lys Asn Ile Pro Ile Gly Phe Trp Trp Ala Val Val Thr Met Thr Thr
385                 390                 395                 400

Leu Gly Tyr Gly Asp Met Tyr Pro Gln Thr Trp Ser Gly Met Leu Val
                405                 410                 415

Gly Ala Leu Cys Ala Leu Ala Gly Val Leu Thr Ile Ala Met Pro Val
            420                 425                 430

Pro Val Ile Val Asn Asn Phe Gly Met Tyr Tyr Ser Leu Ala Met Ala
        435                 440                 445

Lys Gln Lys Leu Pro Lys Lys Lys Lys Lys His Ile Pro Arg Pro Pro
    450                 455                 460

Gln Leu Gly Ser Pro Asn Tyr Cys Lys Ser Val Val Asn Ser Pro His
465                 470                 475                 480

His Ser Thr Gln Ser Asp Thr Cys Pro Leu Ala Gln Glu Glu Ile Leu
                485                 490                 495

Glu Ile Asn Arg Ala Asp Ser Lys Leu Asn Gly Glu Val Ala Lys Ala
            500                 505                 510

Ala Leu Ala Asn Glu Asp Cys Pro His Ile Asp Gln Ala Leu Thr Pro
        515                 520                 525

Asp Glu Gly Leu Pro Phe Thr Arg Ser Gly Thr Arg Glu Arg Tyr Gly
    530                 535                 540

Pro Cys Phe Leu Leu Ser Thr Gly Glu Tyr Ala Cys Pro Pro Gly Gly
545                 550                 555                 560

Gly Met Arg Lys Asp Leu Cys Lys Glu Ser Pro Val Ile Ala Lys Tyr
                565                 570                 575

Met Pro Thr Glu Ala Val Arg Val Thr
            580                 585

<210> 3
<211> 45
<212> DNA
<213> Artificial Sequence

<220>

<223> oligonucleotide (45mer) used for in situ hybridization for the Kv
3.1 alpha-subunit mRNA

<400> 3
atagtcgaag tggctgtggg cgtccggctc cgccagccag gcaag 45